# DEMANDE DE BREVET EUROPEEN

(11) **EP 3 431 286 A1**
(43) Date de publication de la demande: **23.01.2019**
(21) Numéro de dépôt: 18179971.9
(22) Date de dépôt: 26.06.2018
(51) Int. Cl.: B32B 37/14, B32B 37/10, B32B 27/40

(54) **MEMBRANE BARRIÈRE FLEXIBLE ET PROCÉDÉ DE FABRICATION DE LA MEMBRANE BARRIÈRE FLEXIBLE**

(30) Priorité: 19.07.2017 FR 1756847
(71) Demandeur: CARMAT, 78140 Vélizy Villacoublay (FR)
(72) Inventeur: GRIMMÉ, Marc, 91120 Palaiseau (FR); DUBUIS, Pascal, 63720 Clerlande (FR); KOUN, Malys-Claire, 91400 Orsay (FR); MARAIS, Stéphane, 76770 Malaunay (FR); MARCANO ZERPA, Aracelys Maria, 76000 Rouen (FR); FATYEYEVA, Kateryna, 76770 Houppeville (FR); PONCIN-ÉPAILLARD, Fabienne, 72000 Le Mans (FR)
(74) Mandataire: Gevers & Orès

(57) **Abrégé**

- Membrane barrière flexible et procédé de fabrication de la membrane barrière flexible.
- La membrane barrière flexible (1) comprend au moins une première couche (2a) de polyuréthane, et au moins une deuxième couche (20) de matériau(x) intégrée à la première couche (2a) de polyuréthane par une modification d'au moins une surface de la ou des couches de polyuréthane. Le ou les matériaux de la ou des deuxièmes couches est compris parmi les matériaux comprenant l'oxyde de silicium inorganique, l'oxyde de silicium organique et un polymère barrière. L'invention concerne également un procédé de fabrication de la membrane barrière flexible (1).

## Description

### DOMAINE TECHNIQUE

La présente invention concerne, de manière générale, le domaine des matériaux ayant des propriétés barrières aux fluides et aux gaz. Plus particulièrement, elle concerne une membrane flexible possédant des propriétés barrières.

### ÉTAT DE LA TECHNIQUE

Les polyuréthanes sont des polymères qui jouent un rôle majeur dans le développement de nombreux dispositifs dans divers domaines, par exemple, dans le domaine des textiles, du bâtiment ou des dispositifs médicaux allant des cathéters aux prothèses (coeur artificiel). Il existe un intérêt croissant pour l'utilisation de polyuréthanes pour les dispositifs médicaux en raison de leurs propriétés mécaniques élevées et réglables, de leur facilité de traitement et principalement de leur bonne biocompatibilité. Cependant, comme la plupart des polymères, ils sont connus pour être facilement perméables à l'eau et au gaz, à cause de leur état amorphe caoutchouteux. La perméabilité aux gaz (et en particulier au dioxygène) et à l'eau du matériau est un facteur important puisque l'entrée d'air à travers une membrane de polyuréthane dans une prothèse se traduirait par une perte d'efficacité. En outre, l'entrée d'eau à travers la membrane peut entraîner la corrosion de parties métalliques intégrées dans la prothèse.

Pour ces raisons, il est essentiel d'utiliser un matériau présentant des propriétés barrières élevées. Les propriétés barrières d'un matériau correspondent à la capacité du matériau à ralentir ou à empêcher le passage de gaz ou de fluides (liquides) à travers lui-même sous l'effet d'un gradient. De façon non limitative, le gradient peut être un gradient de pression, un gradient de concentration et/ou un gradient de température. Plus les propriétés barrières sont élevées, plus le gaz ou le fluide est ralenti lors de son passage à travers le matériau. Dans l'état de la technique, il existe différentes approches pour accroître les propriétés barrières des polyuréthanes au gaz et à l'eau. Ces voies d'accroissement peuvent être divisées en deux grandes familles : la modification en volume du polyuréthane ou le traitement de surface du polyuréthane.

Dans le cas de la modification en volume, il existe la voie des mélanges de polymères qui permet de contrôler aisément les propriétés de transport des gaz en ajustant le rapport de composition et la microstructure des polymères constitutifs du mélange. La modification par mélange de polymères fait l'objet d'un intérêt particulier de la part des industriels, à la fois pour sa simplicité de mise en oeuvre et pour la stabilité entre les phases, à la différence des systèmes multicouches qui sont parfois le siège de phénomènes de délamination. La voie des mélanges permet également l'élaboration de matériaux plus performants. Toutefois, elle n'est applicable qu'à la condition que les polymères choisis soient miscibles ou présentent une compatibilité suffisante entre eux.

Une autre voie de modification en volume consiste en l'incorporation de nanoparticules dans la matrice polyuréthane pour former un nanocomposite. Les nanocomposites sont des matériaux qui résultent de l'incorporation de nanostructures ou nanocharges dans une matrice polymère organique. Contrairement aux composites usuels où les charges dispersées sont de taille micrométrique, cette incorporation de nanocharges conduit à une augmentation des interfaces et des interactions spécifiques menant à une forte modification des propriétés globales de matériaux. Cette approche s'est révélée être prometteuse pour améliorer les propriétés barrières des matériaux. L'amélioration des propriétés barrières est généralement attribuée à une augmentation de la tortuosité. Dans le cas des nanocomposites, les nanocharges étant considérées comme imperméables, le chemin de diffusion des petites molécules est allongé ce qui entraîne une diminution de la perméabilité du matériau. Cet effet est d'autant plus important que les nanocharges sont de structures lamellaires ou sphériques et si possible orientées perpendiculairement au chemin de diffusion. Du point de vue industriel, l'intérêt porté sur ces matériaux nanocomposites est basé sur le faible coût des argiles à partir desquelles sont issues les nanocharges, la facilité de mise en oeuvre par des procédés usuels (injection-soufflage, extrusion,...) et l'amélioration significative des propriétés fonctionnelles. Cependant, pour des applications biomédicales, il faut s'assurer de la biocompatibilité de charges ajoutées à la matrice polyuréthane et de l'absence de risque de migrations des nanocharges à la surface des matériaux par effet d'élution.

Dans le cas du traitement en surface, une approche décrite dans l'état de la technique pour améliorer les propriétés barrières consiste à appliquer sur le substrat polymère des revêtements permettant de réduire la perméabilité globale des matériaux traités. Cette méthode permet de combiner les propriétés de chacune des couches déposées. Il existe de nombreux exemples commerciaux de matériaux multicouches, en particulier pour des applications d'emballages alimentaires. Outre les techniques consistant à réaliser des structures en sandwich, il est également possible de déposer de couches externes ou internes sur le polymère permettant d'améliorer ses propriétés barrières.

Compte tenu des contraintes physico-chimiques et biologiques imposées par l'utilisation biomédicale des membranes à base de polyuréthane, en particulier les prothèses cardiaques, ces techniques ne semblent pas les plus appropriées pour accroître les propriétés barrières du polyuréthane à la fois aux gaz (au dioxygène en particulier) et à l'eau sans en altérer ses propriétés et la biocompatibilité, et ceci avec le souci de modification de la forme du matériau de géométrie complexe (forme irrégulière en trois dimensions).

### EXPOSÉ DE L'INVENTION

La présente invention a pour objet de pallier ces inconvénients en proposant une membrane flexible ayant des propriétés barrières à l'eau et au dioxygène.

À cet effet, l'invention concerne une membrane barrière flexible.

Selon l'invention, la membrane barrière flexible comprend :
- au moins une première couche de polyuréthane, et
- au moins une deuxième couche de matériau(x) intégrée à la première couche de polyuréthane par une modification d'au moins une surface de la ou des couches de polyuréthane, le ou les matériaux de la ou des deuxièmes couches étant compris parmi les matériaux comprenant l'oxyde de silicium inorganique, l'oxyde de silicium organique de formule chimique SiOₓC_{z}H_{w} et un polymère barrière.

Ainsi, la membrane telle que décrite ci-dessus bénéficie des propriétés barrières à l'eau et au dioxygène apportées par la couche de matériau(x) obtenue par la modification d'au moins une surface de la couche de polyuréthane.

De plus, le polymère barrière est compris parmi les polymères suivants : le polyéthylène-co-alcool vinylique (EVOH), le polychlorure de vinylidène (PVDC), le polyisobutylène (PIB).

Par ailleurs, avantageusement, la ou les couches de matériau(x) sont prises en sandwich entre deux couches de polyuréthane.

L'invention concerne également un procédé de fabrication d'une membrane barrière flexible.

Selon l'invention, le procédé comprend les étapes suivantes :
- une étape de préparation d'une couche de polyuréthane ;
- une étape de modification d'au moins une surface de la couche de polyuréthane de façon que la ou les surfaces de la couche de polyuréthane forment au moins une couche en matériaux, le ou les matériaux étant compris parmi les matériaux comprenant un polymère barrière, l'oxyde de silicium inorganique et l'oxyde de silicium organique de formule chimique SiOₓC_{z}H_{w}.

En outre, le polymère barrière est compris parmi les polymères suivants : l'EVOH, le PVDC et le PIB.

Selon un premier mode de réalisation, l'étape de modification comprend une modification par coulage-évaporation incluant les sous-étapes suivantes :
- une sous-étape de coulage d'une solution de polyéthylène-co-acétate de vinyle (EVA) sur au moins une surface de la couche de polyuréthane, la sous-étape de coulage permettant de déposer une couche d'EVA sur la couche de polyuréthane ;
- une sous-étape d'hydrolyse de la couche d'EVA pour obtenir une couche d'EVOH.

Selon un deuxième mode de réalisation, l'étape de modification comprend une modification par jet de solution pulvérisée incluant les sous-étapes suivantes :
- une sous-étape de préparation d'une solution contenant au moins un polymère barrière ;
- une sous-étape de projection consistant à projeter ladite solution sur au moins une surface de la couche de polyuréthane ;
- une sous-étape de séchage pour former une couche comprenant au moins un polymère barrière.

Selon une première variante d'un troisième mode de réalisation, l'étape de modification comprend une première modification par dépôt chimique en phase vapeur assisté par plasma incluant les sous-étapes suivantes :
- une sous-étape de mise en place de la couche de polyuréthane dans un milieu sous vide d'air ;
- une sous-étape d'introduction d'un précurseur d'oxyde de silicium sous forme gazeuse dans le milieu sous vide d'air, de façon que le précurseur soit réparti de manière homogène dans le milieu sous vide d'air ;
- une sous-étape d'exposition au précurseur de la couche de polyuréthane afin de former une couche comprenant de l'oxyde de silicium inorganique.

Selon une deuxième variante du troisième mode de réalisation, l'étape de modification comprend une deuxième modification par dépôt chimique en phase vapeur assisté par plasma incluant les sous-étapes suivantes :
- une sous-étape de mise en place de la couche de polyuréthane dans un milieu sous vide d'air ;
- une sous-étape d'introduction de dioxygène et d'un précurseur d'oxyde de silicium sous forme gazeuse dans le milieu sous vide d'air, de façon que le dioxygène et le précurseur soit répartis de manière homogène dans le milieu sous vide d'air ;
- une sous-étape d'exposition au dioxygène et au précurseur de la couche de polyuréthane afin de former une couche comprenant de l'oxyde de silicium organique.

De plus, le précurseur d'oxyde de silicium comprend un précurseur organosilicié sous forme gazeuse.

À titre d'exemple, le précurseur organosilicié est compris parmi les précurseurs suivants : le tétraméthylsilane (TMS), le tétraéthyloxisilane, l'hexaméthyldisiloxane, l'hexaméthyldisilazane, le tétraéthylsilane, le tétraméthyldisilazane, le tétraméthyle orthosilicate et le tétraméthylcyclotétrasiloxane.

Selon une variante, le précurseur d'oxyde de silicium comprend un précurseur hydrocarboné.

À titre d'exemple, le précurseur hydrocarboné correspond à l'acétylène.

Par ailleurs, le procédé comprend en outre une étape de dépôt d'au moins une deuxième couche de polyuréthane sur la ou les couches de matériaux de façon que la ou les couches de matériaux soient prises en sandwich entre les deux couches de polyuréthane.

### BRÈVE DESCRIPTION DES FIGURES

L'invention, avec ses caractéristiques et avantages, ressortira plus clairement à la lecture de la description faite en référence aux dessins annexés dans lesquels :
- la figure 1 représente un mode de réalisation de la membrane barrière flexible obtenue à partir d'une modification de la couche de polyuréthane par coulage-évaporation,
- la figure 2 représente un autre de mode de réalisation de la membrane barrière flexible obtenue à partir d'une modification de la couche de polyuréthane par coulage-évaporation,
- la figure 3 représente un mode de réalisation de la membrane barrière flexible obtenue à partir d'une modification de la couche de polyuréthane par dépôt chimique en phase vapeur assisté par plasma,
- les figures 4 à 6 représentent un mode de réalisation de la membrane barrière flexible obtenue à partir d'une modification de la couche de polyuréthane par jet de solution d'EVOH pulvérisée à des grades d'EVOH différents,
- la figure 7 représente un mode de réalisation de la membrane barrière flexible obtenue à partir d'une modification de la couche de polyuréthane par jet de solution de PVDC pulvérisée,
- la figure 8 représente un mode de réalisation de la membrane barrière flexible obtenue à partir d'une modification de la couche de polyuréthane par jet de solution de PIB pulvérisée,
- la figure 9 représente un mode de réalisation de la membrane barrière flexible dans laquelle les couches de matériau sont comprises entre deux couches de polyuréthane, chacune des couches de matériau étant obtenue par modification des couches de polyuréthane par jet de solution de PVDC et d'EVOH,
- la figure 10 représente un mode de réalisation de la membrane barrière flexible dans laquelle la couche de matériau est comprise entre deux couches de polyuréthane, la couche de matériau étant obtenue par une modification des couches de polyuréthane par jet de solution de PVDC,
- la figure 11 représente un mode de réalisation de la membrane barrière flexible dans laquelle les couches de matériau sont comprises entre deux couches de polyuréthane, chacune des couches de matériau étant obtenue par modification des couches de polyuréthane par jet de solution de PVDC et de PIB,
- la figure 12 représente un mode de réalisation de la membrane barrière flexible dans laquelle les couches de matériau sont comprises entre deux couches de polyuréthane, une des couches de matériau étant obtenue par modification par jet de solution de PVDC, les autres couches étant obtenues par modification par dépôt chimique en phase vapeur assisté par plasma,
- la figure 13 représente schématiquement les principales étapes du procédé de fabrication de la membrane barrière flexible.

### DESCRIPTION DÉTAILLÉE

La suite de la description fera référence aux figures citées ci-dessus.

L'invention concerne une membrane barrière flexible 1.

Ladite membrane barrière flexible 1 comprend au moins une première couche 2a de polyuréthane.

Pour une application biomédicale, le polyuréthane composant la membrane barrière flexible 1 peut être n'importe quel type de polyuréthane ayant des propriétés mécaniques, de biostabilité et de biocompatibilité requises. À titre d'exemple, le polyuréthane correspond à un polycarbonate-uréthane ou un polycarbonate-uréthane-urée. Ces polyuréthanes peuvent être également connus, respectivement, sous les noms commerciaux *« Bionate®II »* de la société DSM Polymère Technology Group et *« Chronoflex®AR-LT*» de la société AdvanSource Biomaterial. Le polyuréthane peut aussi être obtenu par synthèse.

La membrane barrière flexible 1 comprend également au moins une deuxième couche 20 de matériau ou matériaux (ci-après, matériau(x)). La deuxième couche 20 de matériau(x) est intégrée à la première couche 2a de polyuréthane par une modification d'au moins une surface de la ou des couches 2a de polyuréthane.

Le ou les matériaux de la ou des deuxièmes couches 20 est compris parmi les matériaux comprenant l'oxyde de silicium inorganique, l'oxyde de silicium organique et un polymère barrière.

Le polymère barrière signifie qu'il s'agit d'un polymère qui possède des propriétés physiques, chimiques ou physicochimiques qui contribuent à réduire le passage de gaz ou d'eau à travers ledit polymère barrière.

Le polymère barrière peut être un polymère synthétisé ou obtenu commercialement.

Le polymère barrière peut être un polymère compris parmi les polymères suivants : le polyéthylène-co-alcool vinylique (EVOH), le polychlorure de vinylidène (PVDC), le polyisobutylène (PIB).

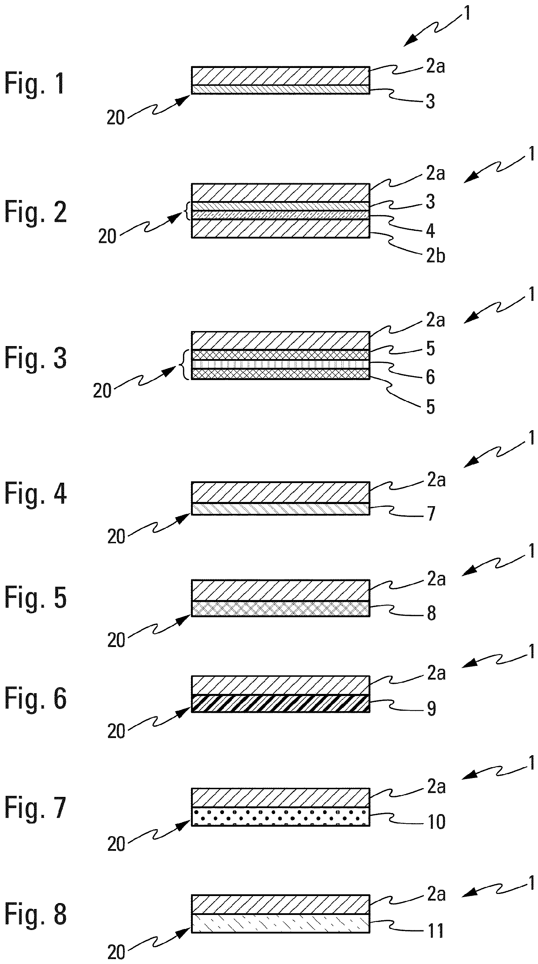

Les figures 4 à 6 représentent une membrane barrière flexible 1 comprenant une couche 2a de polyuréthane et une couche 7, 8, 9 d'EVOH.

La figure 7 représente une membrane barrière flexible 1 comprenant une couche 2a de polyuréthane et une couche 10 de PVDC.

La figure 8 représente une membrane barrière flexible 1 comprenant une couche 2a de polyuréthane et une couche 11 de PIB.

Par exemple, l'EVOH et le PIB proviennent de la société Sigma Aldrich et le PVDC de la société Goodfellow Cambridge Limited.

Il est possible d'utiliser différents grades d'EVOH. Par exemple, un EVOH avec 27% de groupements éthylènes (EVOH27), un EVOH avec 32% de groupements éthylènes (EVOH32) ou un EVOH avec 44% de groupements éthylènes (EVOH44).

La figure 4 représente une membrane barrière flexible 1 comprenant une couche 2a de polyuréthane et une couche 7 d'EVOH27.

La figure 5 représente une membrane barrière flexible 1 comprenant une couche 2a de polyuréthane et une couche 8 d'EVOH32.

La figure 6 représente une membrane barrière flexible 1 comprenant une couche 2a de polyuréthane et une couche 9 d'EVOH44.

L'oxyde de silicium inorganique a pour formule chimique : SiOₓ, dans laquelle le paramètre x de l'oxyde de silicium inorganique représente le rapport entre la quantité d'oxygène et la quantité de silicium. De façon non limitative, le paramètre x est compris entre 1,5 et 2,2, de préférence entre 1,8 et 2,1.

L'oxyde de silicium organique a pour formule chimique : SiOₓC_{z}H_{w}. De façon non limitative, le paramètre x de l'oxyde de silicium organique est compris entre 1 et 2,4, le paramètre z étant compris entre 0,2 et 2,4, le paramètre w étant compris entre 0,05 et 4.

Selon un mode de réalisation, la ou les couches 20 de matériau(x) sont prises en sandwich entre deux couches 2a et 2b de polyuréthane.

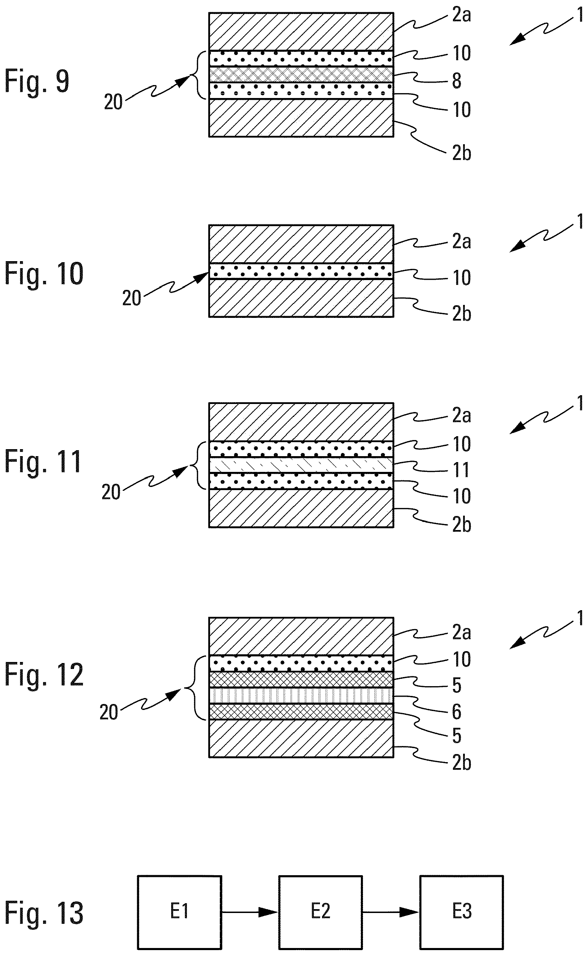

La membrane barrière flexible 1 est fabriquée à partir d'un procédé comprenant les étapes suivantes (figure 13) :
- une étape E1 de préparation d'une couche 2a de polyuréthane ;
- une étape E2 de modification d'au moins une surface de la couche 1 polyuréthane de façon que la ou les surfaces de la couche 2a de polyuréthane forment au moins une couche 20 en matériaux, le ou les matériaux étant compris parmi les matériaux comprenant l'oxyde de silicium inorganique, l'oxyde de silicium organique et un polymère barrière.

Selon un premier mode de réalisation (figures 1 et 2), l'étape E2 de modification comprend une modification par coulage-évaporation afin d'obtenir une couche d'EVOH.

L'étape de modification comprend alors une sous-étape de coulage d'une solution de polyéthylène-co-acétate de vinyle (EVA) sur au moins une surface de la couche 2 de polyuréthane. Cette sous-étape de coulage permet de déposer une couche d'EVA 4 sur la couche de polyuréthane.

L'EVA utilisé est, par exemple, un copolymère à 70% en masse d'acétate de vinyle également connu sous le nom de Levapren® 700 de la société Lanxess Co. dont la masse molaire Mw est sensiblement égale à 268 000 g/mol.

La solution d'EVA a été préparée à partir d'au moins un des solvants suivants : le chloroforme, le dichlorométhane, le diméthyacétamide (DMAc) et le diméthylformamide (DMF).

L'étape E2 de modification comprend également une sous-étape d'hydrolyse de la couche d'EVA 4 après évaporation du ou des solvants pour obtenir une couche d'EVOH 3. L'hydrolyse correspond à une hydrolyse de surface de l'EVA à l'aide d'une solution basique (NaOH par exemple) et en présence d'eau. L'épaisseur de la couche 3 hydrolysée varie en fonction du temps de traitement (figures 1 et 2).

Selon un deuxième mode de réalisation (figures 4 à 12), l'étape E2 de modification comprend une modification par jet de solution pulvérisée.

L'étape E2 de modification comprend alors une sous-étape de préparation d'une solution contenant au moins un polymère barrière inclus parmi les matériaux comprenant l'EVOH, le PVDC, le PIB. La solution est préparée à partir d'au moins un des polymères barrière et d'un solvant.

L'étape E2 de modification comprend une sous-étape de projection consistant à projeter ladite solution sur au moins une surface de la couche 2a de polyuréthane.

La concentration de polymère barrière dans la solution est telle que la solution soit suffisamment fluide pour le bon fonctionnement d'un aérographe permettant le jet de solution pulvérisée et pour obtenir des couches homogènes et uniformes. Par exemple, l'aérographe est un aérographe du modèle Fengda® BD-134K. L'aérographe permet de pulvériser la solution sous forme d'aérosol de manière continue et homogène sur la surface à modifier. Par exemple, la solution contient entre 3% et 20% en masse de polymère barrière à pulvériser sur la ou les surfaces de la couche 2a en polyuréthane l'aide d'un aérographe. Pour cela, une quantité prédéterminée de polymère a été dissoute dans du DMAc sous agitation afin de former une solution homogène (10%m/m pour des EVOH de grades différents et 20%m/m pour le PVDC). Dans le cas du PIB, le m-xylène (ou méta-xylène) a été utilisé comme solvant (3 %m/m). Puis, la solution a été versée dans le godet de l'aérographe pour être ensuite pulvérisée, avec une pression horizontale et verticale maximale, à la surface de la ou des surfaces de la couche 2a en polyuréthane préalablement nettoyées à l'air comprimé pour éliminer la poussière. Le revêtement a été réalisé sous forme de monocouche et multicouches.

L'étape de modification comprend une sous-étape de séchage pour former une couche 4, 5, 6, 7, 8 qui comprend au moins un polymère barrière. De manière non limitative, le séchage dure environ dix minutes.

La sous-étape de projection et la sous-étape de séchage peuvent être réalisées plusieurs fois avec différents polymères barrières. Lorsque toutes les couches 20 sont réalisées par ces sous-étapes, la membrane est séchée pendant 48 heures à une température de 60°C dans une étuve thermorégulée.

Selon une première variante d'un troisième mode de réalisation (figure 3), l'étape E2 de modification comprend une première modification par dépôt chimique en phase vapeur assisté par plasma (ou PECVD pour « Plasma-Enhanced Chemical Vapor Déposition »).

La première modification comprend alors :
- une sous-étape de mise en place de la couche 2a de polyuréthane dans un milieu sous vide d'air ;
- une sous-étape d'introduction d'un précurseur d'oxyde de silicium sous forme gazeuse dans le milieu sous vide d'air, de façon que le précurseur soit réparti de manière homogène dans le milieu sous vide d'air ;
- une sous-étape d'exposition au précurseur de la couche 2a de polyuréthane afin de former une couche 5 comprenant de l'oxyde de silicium inorganique.

Selon une deuxième variante du troisième mode de réalisation, l'étape de modification comprend une deuxième modification par la technique PECVD.

La deuxième modification comprend alors :
- une sous-étape de mise en place de la couche 2a de polyuréthane dans un milieu sous vide d'air ;
- une sous-étape d'introduction de dioxygène et d'un précurseur d'oxyde de silicium sous forme gazeuse dans le milieu sous vide d'air, de façon que le dioxygène et le précurseur soit répartis de manière homogène dans le milieu sous vide d'air ;
- une sous-étape d'exposition au dioxygène et au précurseur de la couche 2a de polyuréthane afin de former une couche 6 comprenant de l'oxyde de silicium organique.

Pour le troisième mode de réalisation, le précurseur d'oxyde de silicium sous forme gazeuse utilisé par la technique PECVD peut inclure un précurseur organosilicié.

À titre d'exemple, le précurseur organosilicié est compris parmi les précurseurs suivants : le tétraméthylsilane (TMS), le tétraéthyloxisilane, l'hexaméthyldisiloxane, l'hexaméthyldisilazane, le tétraéthylsilane, le tétraméthyldisilazane, le tétraméthyle orthosilicate et le tétraméthylcyclotétrasiloxane.

Selon une variante, le précurseur d'oxyde de silicium peut comprendre un précurseur hydrocarboné.

À titre d'exemple, le précurseur hydrocarboné correspond à l'acétylène.

La réalisation de la ou des deuxièmes couches 5, 6 en oxydes de silicium organiques et/ou inorganiques a été mise en oeuvre à l'aide d'un dispositif PECVD. Par exemple, le dispositif PECVD est composé d'un réacteur radiofréquence équipé d'un générateur d'ondes électromagnétiques à radiofréquence de 13,6 MHz (par exemple, un générateur Sairem 0-600 W) et d'une enceinte en aluminium sous forme d'un parallélépipède possédant une cathode rectangulaire (par exemple, de dimensions 100 mmx210 mm) reliée au générateur et une anode rectangulaire (par exemple, de dimensions 100 mmx210 mm) reliée à la terre. Une fois la couche de polyuréthane introduite dans le réacteur, un vide primaire, allant jusqu'à 10⁻⁵ mbar, et puis un vide secondaire, avoisinant les 10⁻⁷ mbar, ont été créés pour éliminer les gaz résiduels susceptibles de polluer le processus. La couche est déposée sur le porte-substrat et le précurseur (par exemple, le TMS) est ensuite introduit sous forme gazeuse dans l'enceinte du réacteur. L'introduction et la distribution du précurseur se font de manière homogène dans tout le volume du réacteur.

Les deux variantes du troisième mode de réalisation peuvent être combinées. Ainsi, des réalisations de multicouches peuvent être mises en oeuvre sur une des surfaces de la couche 2a de polyuréthane à partir d'un précurseur d'oxyde de silicium sous forme gazeuse (par exemple, le TMS), en changeant à des instants donnés le rapport entre le précurseur et le dioxygène dans l'enceinte du dispositif PECVD. Par exemple, trois couches successives 5, 6, 5 sont réalisées en alternants des couches d'oxyde de silicium organique (SiOₓC_{z}H_{w}) 6 et inorganique (SiOₓ) 5. Une première série peut être réalisée en introduisant dans l'enceinte du précurseur seul, en introduisant ensuite un mélange de précurseur et de dioxygène, puis en introduisant encore du précurseur seul (TMS/TMS+O₂/TMS). Une deuxième série peut également être réalisée en introduisant un mélange de précurseur et de dioxygène, en introduisant ensuite du précurseur seul, puis en introduisant encore un mélange de précurseur et de dioxygène (TMS+O₂/TMS/TMS+O₂). De façon non limitative, les deux séries peuvent être réalisées sur au moins une des surfaces de la couche 2a de polyuréthane avec un temps d'exposition de 8 min par couche, sous une pression de 4,4·10⁻² mbar, avec un débit de dioxygène de 5 sccm (ou 5 cm³/min à des conditions standard de température et de pression) et une puissance d'excitation de 100 W.

Les différents modes de réalisation présentés ci-dessus peuvent être combinés entre eux.

Ainsi, par exemple, la combinaison du troisième mode de réalisation correspondant à une modification par la technologie PECVD et le deuxième mode de réalisation correspondant à une modification par jet de solution pulvérisée permet d'accroître les propriétés barrières au dioxygène et à l'eau de la couche 2a de polyuréthane sans en altérer ses propriétés en volume, sa biostabilité, ni sa biocompatibilité. La perméabilité au dioxygène de la couche 2a de polyuréthane est réduite de 99% et celle de l'eau est réduite à 90%.

Pour évaluer les propriétés barrières de la membrane barrière flexible 1, plusieurs combinaisons ont pu être réalisées.

Le tableau A ci-dessous récapitule des résultats de mesures de perméabilité au dioxygène et à l'eau de membranes barrières flexibles obtenues par différents modes de réalisation du procédé. Le tableau A utilise le Barrer comme unité pour le coefficient de perméabilité. Une valeur de 1 Barrer correspond à une valeur de 1 × 10⁻¹⁰ Ncm³.cm/(cm².s.cmHg).

Des techniques de mesure ont été utilisées telles que l'analyse thermique gravimétrique, l'analyse calorimétrique différentielle, la spectroscopie infrarouge par transformée de Fourier en mode de réflexion totale atténuée (spectroscopie IRFT-ATR), la microscopie électronique à balayage, la microscopie à force atomique, l'angle de contact, la perméabilité à l'eau et la perméabilité au dioxygène.

Les flux de dioxygène sont mesurés en considérant le passage de dioxygène depuis une surface extérieure de la couche 2a de polyuréthane non modifiée vers l'intérieur. Par exemple, les mesures de perméabilité au dioxygène sec sont réalisées avec l'aide d'un OX-TRAN Model 2/21 de la société Mocon Inc. Il est composé de deux cellules de mesure et d'un détecteur coulométrique dit ultrabasse trace COULOX® pour tester des matériaux fortement barrières au dioxygène. Chaque cellule est composée de deux compartiments, dénommés chambre extérieure et chambre intérieure, séparées par la membrane barrière flexible 1 à tester. Chaque cellule est balayée par un gaz inerte constitué d'un mélange de diazote et de dihydrogène (N₂ à 95%) afin d'éliminer toute trace de dioxygène. Ensuite, une fois le signal stable et proche de zéro, le dioxygène est appliqué à flux constant dans les chambres extérieures des deux cellules. Finalement, le dioxygène ayant traversé la membrane barrière flexible 1 est détecté dans les chambres intérieures des cellules par le capteur ultrabasse trace.

Les flux d'eau sont mesurés en considérant le passage de l'eau depuis une surface extérieure de la couche 2a de polyuréthane non modifiée vers l'intérieur. Les mesures de perméabilité à l'eau liquide ont été réalisées sur un perméamètre composé d'une cellule, comprenant deux compartiments dits amont et aval, entre lesquels la membrane barrière flexible 1 à tester est placée. L'étanchéité est obtenue par l'utilisation de joints disposés autour de la surface active de la membrane barrière flexible 1. Lors de la mesure, l'eau liquide est introduite dans le compartiment amont, tandis que le compartiment aval est balayé par un gaz sec (par exemple, du diazote). Au cours du temps, le gaz balayant le compartiment aval s'enrichit en eau. Un hygromètre à miroir (par exemple, de General Eastern) détecte les molécules d'eau ayant traversé la membrane barrière flexible 1 via la mesure de la température de rosée T_{R}.

Selon une première combinaison, on a réalisé une membrane barrière flexible 1 multicouches par une modification des couches de polyuréthane (par exemple polyuréthane « *Chronoflex®AR-LT*») par coulage-évaporation d'EVA qui a subi une hydrolyse (figures 1 et 2).

Pour cela, une solution d'EVA (mélange d'EVA70 et de chloroforme) a été coulée sur une couche 2a en polyuréthane. La membrane a ensuite été laissée à température ambiante pendant 3 heures puis placée dans une étuve à 60°C pour éliminer le solvant résiduel. Cette couche d'EVA70 est ensuite hydrolysée à partir d'une solution à base de soude (NaOH 0,8M contenant 75%v/v de méthanol et 25% d'eau distillée (« *Milli-Q* »)). La durée d'hydrolyse est de 5 heures. La membrane est ensuite lavée plusieurs fois à l'eau distillée, puis elle est immergée dans une solution 3M d'acide chlorhydrique pendant 15 min. La membrane est ensuite rincée à l'eau distillée « *Milli-Q »* jusqu'à obtention d'un pH neutre et puis séchée. La perméabilité au dioxygène, à 23°C, des couches de polyuréthane modifiées a été réduite jusqu'à 55,3% par rapport à une couche de polyuréthane non modifiée. La perméabilité au dioxygène est donc passée de 2,0 Barrer à 0,92 Barrer.

Selon une deuxième combinaison, on a réalisé une membrane barrière flexible 1 multicouches par une modification par la technologie PECVD sur des couches de polyuréthane (par exemple polyuréthane *« Chronoflex®AR-LT*») (figure 3).

Pour cela, un dépôt tri-couches à partir de TMS/TMS+O₂/TMS a été réalisé sur des couches 2a de polyuréthane avec du TMS comme gaz précurseur à l'aide d'un réacteur radiofréquence équipé d'un générateur de 13,6 MHz (Sairem 0-600 W) travaillant à une puissance de 100 W. Dans le cas du mélange TMS et dioxygène (TMS+O₂), un débit de dioxygène de 5 sccm a été utilisé. Un temps d'exposition de 8 min par couche a été fixé (à une pression de p=4,4·10⁻² mbar). Les membranes modifiées ont présenté une diminution de la perméabilité au dioxygène de 65% par rapport aux membranes non modifiées. La perméabilité au dioxygène est donc passée de 2,0 Barrer à 0,7 Barrer. Les membranes 1 modifiées ont présenté une diminution de la perméabilité à l'eau de 5% par rapport aux membranes non modifiées. La perméabilité au dioxygène est donc passée de 7387 Barrer à 7000 Barrer à 25°C.

Selon une troisième combinaison, on a réalisé une membrane barrière flexible 1 multicouches par une modification par jet de solution pulvérisée d'EVOH et de PVDC sur des couches 2a de polyuréthane (par exemple polyuréthane « *Chronoflex®AR-LT* ») (figure 9).

Une solution d'EVOH32 (32% de taux d'éthylène) a été pulvérisée à la surface des couches 2a de polyuréthane à l'aide d'un aérographe (par exemple un aérographe Fengda® BD-134K) avec un diamètre de buse et d'aiguille de 0,5 mm et une pression de travail constante de 4 bar. Un temps de séchage de 10 min entre chaque couche a été utilisé puis les membranes obtenues ont été séchées 48 heures à 60°C. Le revêtement a été constitué de 6 couches de solution EVOH32 10%m/m, présentant une épaisseur finale après séchage d'environ 20 µm. Les membranes modifiées ont présenté une diminution de la perméabilité au dioxygène jusqu'à 99% à 37°C par rapport aux membranes non modifiées. La perméabilité au dioxygène est donc passée de 3,98 Barrer à 0,04 Barrer. Les membranes 1 modifiées ont présenté une diminution de la perméabilité à l'eau de 54% par rapport aux membranes non modifiées. La perméabilité au dioxygène est donc passée de 8331 Barrer à 3825 Barrer.

Selon une quatrième combinaison, on a réalisé une membrane barrière flexible 1 multicouches par une modification par jet de solution pulvérisée de PVDC et de PIB sur des couches de polyuréthane (par exemple polyuréthane « *Chronoflex®AR-LT*») (figure 11).

Une solution de PVDC 20%m/m a été pulvérisée à la surface des couches de polyuréthane comme décrit dans la troisième combinaison. Le revêtement a été constitué de trois couches de solution PVDC 20%m/m, présentant une épaisseur finale après séchage d'environ 20 µm. Après séchage, ce revêtement a été recouvert avec six couches de solution PIB 3%m/m, présentant une épaisseur finale après séchage d'environ 12 µm. Après séchage, une couche d'environ 6 µm de PVDC 20%m/m a été pulvérisée de façon à assurer l'adhésion entre le PIB et le polyuréthane. Finalement, cette couche a été revêtue avec une solution polyuréthane à 8% avec une épaisseur finale d'environ 12 µm. Les membranes 1 modifiées ont présenté une diminution de la perméabilité au dioxygène jusqu'à 95% à 37°C par rapport aux membranes non modifiées. La perméabilité au dioxygène est donc passée de 3,98 Barrer à 0,19 Barrer. Les membranes 1 modifiées ont présenté une diminution de la perméabilité à l'eau de 90% par rapport aux membranes non modifiées. La perméabilité au dioxygène est donc passée de 8331 Barrer à 848 Barrer.

Selon une cinquième combinaison, on a réalisé une membrane barrière flexible 1 multicouches par une modification par la technologie PECVD et par une modification par jet de solution pulvérisée de PVDC (figure 12).

Une solution de PVDC 20%m/m a été pulvérisée à la surface des couches de polyuréthane (par exemple polyuréthane « *Chronoflex®AR-LT*») à l'aide d'un aérographe (par exemple un aérographe Fengda® BD-134K), ayant un diamètre de buse et d'aiguille de 0,5 mm et une pression constante de travail de 4 bar. Le revêtement PVDC a été constitué de six couches de solution PVDC 20%m/m présentant une épaisseur finale après séchage d'environ 40 µm. Un temps de séchage de 10 min entre chaque couche a été utilisé puis les membranes ont été séchées 48 h à 60°C. Par la suite, un dépôt à trois couches à partir du TMS/TMS+O₂/TMS a été réalisé sur des couches de « *Chronoflex®AR-LT*» à partir du TMS à l'aide d'un réacteur radiofréquence équipé d'un générateur de 13,6 MHz (par exemple un générateur Sairem 0-600 W) travaillant à une puissance de 100 W. Un débit de dioxygène de 5 sccm a été utilisé. Un temps d'exposition de 8 min par couche a été fixé (à une pression de p=4,4·10⁻² mbar). Ce dépôt par plasma a ensuite été revêtu avec une solution de polyuréthane à 8%. Un temps de séchage de 10 minutes entre chaque couche a été utilisé, puis les membranes ont été séchées 48 heures à 60°C. L'épaisseur finale du revêtement de polyuréthane est d'environ 12 µm. Les membranes 1 modifiées ont présenté une diminution de la perméabilité au dioxygène jusqu'à 88% à 37°C par rapport aux membranes non modifiées. La perméabilité au dioxygène est donc passée de 3,98 Barrer à 0,48 Barrer. Les membranes modifiées ont présenté une diminution de la perméabilité à l'eau de 84% à 37°C par rapport aux membranes non modifiées. La perméabilité au dioxygène est donc passée de 8331 Barrer à 1363 Barrer.

Afin de s'assurer une biocompatibilité, d'autres combinaisons ont été réalisées en déposant une couche 2b de polyuréthane supplémentaire prenant en sandwich les couches 20 de matériaux. Ce dépôt de couche 2b de polyuréthane peut être réalisée avec le dépôt d'une solution de polyuréthane.

**Tableau A**

| Membranes | T(°C) | Perméabilité au dioxygène | | | | Perméabilité à l'eau | | | |
|---|---|---|---|---|---|---|---|---|---|
| | | Épaisseur (µm) | Flux (cm³/m³/jour) | Coefficient de perméabilité | | Épaisseur (µm) | Flux ×10⁶ (mmol/cm²/s) | Coefficient de perméabilité | |
| | | | | × 10¹⁰ cm³/cm/s/cmHg | Barrer | | | × 10⁶ cm³/cm/s/cmHg | Barrer |
| Polyuréthane (PU) | 25 | 332,2 | 447,9 | 2,27 | 2,03 | 352,0 | 2,2 | 0,80 | 7466 |
| | 27 | 329,4 | 929,0 | 4,66 | 3,98 | 366,9 | 5,0 | 0,99 | 8331 |

| Modification du PU par coulage-évaporation | | | | | | | | | |
|---|---|---|---|---|---|---|---|---|---|
| PU/EVA hydrolysé | 23 | 540 | 124,1 | 1,02 | 0,92 | | | | |
| PU/EVA hydrolysé/EVA/PU | 23 | 613 | 159,2 | 1,48 | 1,34 | | | | |

| Modification du PU par la technologie PECVD | | | | | | | | | |
|---|---|---|---|---|---|---|---|---|---|
| PU/TMS/TMS+O₂/TMS | 25 | 338,3 | 151,6 | 0,78 | 0,70 | 318,2 | 2,3 | 0,75 | 7000 |
| | 37 | | 959,8 | 4,94 | 4,12 | | 5,1 | 0,88 | 7968 |

| Modification du PU par jet de solution pulvérisée | | | | | | | | | |
|---|---|---|---|---|---|---|---|---|---|
| PU/EVOH27 | 37 | 365,3 | 2,87 | 0,01 | 0,02 | 355,0 | 2,3 | 0,44 | 3933 |
| PU/EVOH32 | 37 | 376,2 | 7,32 | 0,04 | 0,04 | 316,9 | 2,2 | 0,43 | 3825 |
| PU/EVOH44 | 37 | 427,8 | 10,6 | 0,06 | 0,07 | 348,0 | 1,9 | 0,37 | 3356 |
| PU/PVDC | 25 | 414,2 | 42,1 | 0,27 | 0,24 | 378,3 | 0,6 | 0,23 | 2240 |
| | 37 | | | | | 372,0 | 1,9 | 0,38 | 3485 |
| PU/PIB | 25 | 368,4 | 466,7 | 2,62 | 2,25 | 358,4 | 0,74 | 0,27 | 2418 |
| PU/PVCD/EVOH32/PVDC/PU | 37 | 386,2 | 26,1 | 0,15 | 0,13 | 382,0 | 0,88 | 0,18 | 1552 |
| PU/PVDC/PU | 37 | 364,6 | 180,2 | 0,60 | 0,86 | 474,6 | 0,72 | 0,18 | 1572 |
| PU/PVDC/PIB/PVDC/PU | 37 | 413,6 | 34,6 | 0,22 | 0,19 | 435,2 | 0,41 | 0,10 | 848 |
| PU/PVDC/TMS/TMS+O₂/TMS/PU | 37 | 391,6 | 94,4 | 0,56 | 0,48 | 370,0 | 0,78 | 0,16 | 1363 |

## Revendications

1. Membrane barrière flexible,
**caractérisée en ce qu'**elle comprend :
- au moins une première couche (2a) de polyuréthane, et
- au moins une deuxième couche (20) de matériau(x) intégrée à la première couche (2a) de polyuréthane par une modification d'au moins une surface de la ou des couches de polyuréthane, le ou les matériaux de la ou des deuxièmes couches étant compris parmi les matériaux comprenant l'oxyde de silicium inorganique, l'oxyde de silicium organique de formule chimique SiOₓC_{z}H_{w} et un polymère barrière.

2. Membrane selon la revendication 1,
**caractérisée en ce que** le polymère barrière est compris parmi les polymères suivants : le polyéthylène-co-alcool vinylique (EVOH), le polychlorure de vinylidène (PVDC), le polyisobutylène (PIB).

3. Membrane selon l'une quelconque des revendications 1 ou 2, **caractérisée en ce que** la ou les couches (20) de matériau(x) sont prises en sandwich entre deux couches (2a, 2b) de polyuréthane.

4. Procédé de fabrication d'une membrane barrière flexible (1), **caractérisé en ce qu'**il comprend les étapes suivantes :
- une étape (E1) de préparation d'une couche (2a) de polyuréthane ;
- une étape (E2) de modification d'au moins une surface de la couche (2a) de polyuréthane de façon que la ou les surfaces de la couche (2a) de polyuréthane forment au moins une couche (20) en matériaux, le ou les matériaux étant compris parmi les matériaux comprenant un polymère barrière, l'oxyde de silicium inorganique et l'oxyde de silicium organique de formule chimique SiOₓC_{z}H_{w}.

5. Procédé selon la revendication 4,
**caractérisé en ce que** le polymère barrière est compris parmi les polymères suivants : l'EVOH, le PVDC et le PIB.

6. Procédé selon l'une quelconque des revendications 4 ou 5,
**caractérisé en ce que** l'étape (E2) de modification comprend une modification par coulage-évaporation incluant les sous-étapes suivantes :
- une sous-étape de coulage d'une solution de polyéthylène-co-acétate de vinyle (EVA) sur au moins une surface de la couche (2a) de polyuréthane, la sous-étape de coulage permettant de déposer une couche d'EVA (3, 4) sur la couche (2a) de polyuréthane ;
- une sous-étape d'hydrolyse de la couche d'EVA pour obtenir une couche (7, 8, 9) d'EVOH.

7. Procédé selon l'une quelconque des revendications 4 à 6,
**caractérisé en ce que** l'étape (E2) de modification comprend une modification par jet de solution pulvérisée incluant les sous-étapes suivantes :
- une sous-étape de préparation d'une solution contenant au moins un polymère barrière ;
- une sous-étape de projection consistant à projeter ladite solution sur au moins une surface de la couche (2a) de polyuréthane ;
- une sous-étape de séchage pour former une couche (7, 8, 9, 10, 11) comprenant au moins un polymère barrière.

8. Procédé selon l'une quelconque des revendications 4 à 7,
**caractérisé en ce que** l'étape (E2) de modification comprend une première modification par dépôt chimique en phase vapeur assisté par plasma incluant les sous-étapes suivantes :
- une sous-étape de mise en place de la couche (2a) de polyuréthane dans un milieu sous vide d'air ;
- une sous-étape d'introduction d'un précurseur d'oxyde de silicium sous forme gazeuse dans le milieu sous vide d'air, de façon que le précurseur soit réparti de manière homogène dans le milieu sous vide d'air ;
- une sous-étape d'exposition au précurseur de la couche de polyuréthane afin de former une couche (5) comprenant de l'oxyde de silicium inorganique.

9. Procédé selon l'une quelconque des revendications 4 à 8,
**caractérisé en ce que** l'étape (E2) de modification comprend une deuxième modification par dépôt chimique en phase vapeur assisté par plasma incluant les sous-étapes suivantes :
- une sous-étape de mise en place de la couche (2a) de polyuréthane dans un milieu sous vide d'air ;
- une sous-étape d'introduction de dioxygène et d'un précurseur d'oxyde de silicium sous forme gazeuse dans le milieu sous vide d'air, de façon que le dioxygène et le précurseur soit répartis de manière homogène dans le milieu sous vide d'air ;
- une sous-étape d'exposition au dioxygène et au précurseur de la couche de polyuréthane afin de former une couche (6) comprenant de l'oxyde de silicium organique.

10. Procédé selon l'une quelconque des revendications 8 ou 9,
**caractérisé en ce que** le précurseur d'oxyde de silicium comprend un précurseur organosilicié sous forme gazeuse.

11. Procédé selon la revendication 10,
**caractérisé en ce que** le précurseur organosilicié est compris parmi les précurseurs suivants : le tétraméthylsilane (TMS), le tétraéthyloxisilane, l'hexaméthyldisiloxane, l'hexaméthyldisilazane, le tétraéthylsilane, le tétraméthyldisilazane, le tétraméthyle orthosilicate et le tétraméthylcyclotétrasiloxane.

12. Procédé selon l'une quelconque des revendications 8 ou 9,
**caractérisé en ce que** le précurseur d'oxyde de silicium comprend un précurseur hydrocarboné.

13. Procédé selon la revendication 12,
**caractérisé en ce que** le précurseur hydrocarboné correspond à l'acétylène.

14. Procédé selon l'une quelconque des revendications 4 à 13,
**caractérisé en ce qu'**il comprend en outre une étape (E3) de dépôt d'au moins une deuxième couche (2b) de polyuréthane sur la ou les couches (20) de matériaux de façon que la ou les couches (20) de matériaux soient prises en sandwich entre les deux couches (2a, 2b) de polyuréthane.
